# EUROPEAN PATENT SPECIFICATION

(11) **EP 1 082 146 B1**
(45) Date of publication and mention of the grant of the patent: **13.04.2005**
(21) Application number: 99915933.8
(22) Date of filing: 14.04.1999
(51) Int. Cl.: A61L 15/26, C08G 18/10

(54) **POLYURETHANE FOAMS FOR USE IN WOUND DRESSINGS**
POLYURETHANSCHÄUME FÜR VERWENDUNG IN WUNDVERBÄNDEN
MOUSSES EN POLYURETHANNE UTILISEES AVEC DES PANSEMENTS

(30) Priority: 15.04.1998 GB 9808006
(43) Date of publication of application: 14.03.2001
(73) Proprietor: Johnson & Johnson Medical Ltd., Edinburgh EH2 4NH (GB)
(72) Inventor: ADDISON, Deborah, Lancaster LA2 8HB (GB); BLACKLOCK, Tracy, West Yorkshire BD22 0AX (GB)
(74) Representative: James, Anthony Christopher W.P.
(86) International application number: PCT/GB1999/001131
(87) International publication number: WO 1999/052569

(56) References cited:
- EP-A- 0 335 669
- EP-A- 0 541 391
- WO-A-88/02636
- DATABASE WPI Week 8750 Derwent Publications Ltd., London, GB; AN 350852 XP002109036 "Manufacturing urethane foam having high deodorizing efficiency" & JP 62 252413 A (BRIDGESTONE TIRE), 4 November 1987 (1987-11-04)
- DATABASE WPI Week 8529 Derwent Publications Ltd., London, GB; AN 176399 XP002109037 "Antiseptic foam composition for applying to wounds" & SU 884 189 A (RIGA TRAUMA ORTHOPA), 23 February 1985 (1985-02-23)

## Description

This invention relates to polyurethane foams, and more particularly to a method of making conformable, high-density polyurethane foams for use in wound dressings. The invention also relates to a wound dressing having a wound-contacting layer formed from such a foam.

Polyurethane foams have been proposed for a number of uses in the prior art. For example, US-A-3903232 discloses hydrophilic cross-linked polyurethane foams which are said to be useful for external body cleaning, for internal body usage and as absorptive products such as diapers. The foams are prepared by reacting particular isocyanate-capped polyoxyethylene polyols having an isocyanate functionality greater than 2 with large amounts of an aqueous reactant, preferably water.

EP-A-0171268 discloses a dressing for use in deep wounds, which dressing comprises individual pieces of absorbent hydrophilic foam contained within a porous bag formed from an apertured polymeric film. The absorbent foam is preferably a hydrophilic polyurethane foam which can be made from HYPOL (Registered Trade Mark) isocyanate-capped polyether prepolymer marketed by W.R. Grace & Co., and nonionic surfactants.

According to EP-A-0171268, the fact that the foam is present in the form of individual pieces confers on the dressing the property of being able to conform to the contours of a wound cavity both on initial application of the dressing and subsequently following absorption of body fluids. It is said that existing commercially available foams, if used as a single piece, have too high a density to possess the required degree of conformability.

US-A-4339550 discloses a hydrophilic foam composition which is prepared by the "in situ" reaction of an isocyanate-capped polyether prepolymer having a functionality of from about. 2 to about 8, water, and a chemically compatible, essentially non-polar, volatile organic compound. The foam is stated to be capable of achieving a sustained, controlled release of the volatile materials from the foamed structure. Suitable "control - release" ingredients include polyols, such as propylene glycol and glycerine.

EP-A-0335669 discloses a hydrophilic foam composition comprising the "in situ" reaction product, of an isocyanate-capped polyether prepolymer, a hydrophilic agent capable of absorbing water, an adjuvant comprising an alcohol, a wetting agent and water. One application which is proposed for the foam composition is in the manufacture of wound dressings. The composition is said to carry the adjuvant releasably, so that at least a portion of the adjuvant is released into an external liquid (e.g. wound exudate) with which the foam composition comes into contact.

. A wide range of prepolymer, hydrophilic agents, adjuvants and wetting agents are proposed in EP-0335669. Suitable prepolymers are said to include prepolymers having an NCO content as high as 2.55 meq/g or as low as 0.5 to 0.9 meq/g. Water soluble monohydric, dihydric and polyhydric alcohols are all said to be suitable adjuvants, but glycerol is preferred, and the majority of the examples involve the use of glycerol. The sole example in which a monohydric alcohol is employed as the adjuvant involves the use of a prepolymer having an NCO content of 1.6 meq/g. The resulting product is said not to be acceptable because of "gross porosity".

EP-A-0541391 polyurethane foam suitable for use as a wound-contacting layer, the method comprising mixing 1 part by weight of an isocyanate-capped prepolymer having from 0.5 to 1.2 meq NCO groups/g with from 0.4 to 1.0 parts by weight of water in the presence of from 0.05 to 0.4 parts by weight of a C₁ to C₃ monohydric alcohol, and then drying the product such that the amount of water-soluble alcohol remaining in the product is less than 1% by weight. The resulting foam has high density and conform ability.

JP-A-62252413 describes the manufacture of a polyurethane foam having high deodorizing efficiency for use in air filters by reacting an isocyanate prepolymer with water in the presence of a deodorizer comprising ferric salts and ascorbic acid.

WO88/02636 describes water hardenable splinting bandages comprising a flexible fabric carrying a water curable isocyanate terminated prepolymer and a catalyst therefor, wherein the prelpolymer is in admixture with an organic acid anhydride to inhibit premature setting of the bandage on storage.

SU-A-884189 describes an antiseptic foam composition for the treatment of wounds containing stearic acid, glycerol, triethanolamine, a propellant, lanolin and other components.

WO99/02587 describes compressed hydrophilic polyurethane foams prepared by reacting an isocyanate prepolymer with warm water. The prepolymer may stabilized during storage by the addition of small quantities of acids.

In certain circumstances it can be desirable to extend the curing time of polyurethane foams such as those described above, for example to allow time for spreading of the polymer mixture to form layers, and to control the density and porosity of the foam product. Extending the cure time prevents premature curing prior to spreading, which may cause foam faults such as spreading lines or holes.

EP-A-0063044 describes a method of extending the curing time of polyurethane gel prepolymers used in soil stabilization. The method comprises stabilizing the water of the prepolymer composition in micelles. EP-A-0063044 notes that the addition of acid also prolongs the curing time of gel prepolymers, but results in product gels having undesirable physical properties. There is no mention of foam polyurethane, or the use thereof in wound dressings.

It has now been found that the addition of pharmaceutically acceptable acids to prepolymer mixtures of the kind described in EP-A-0541391 results in extended curing times without any deterioration in the physical properties of the resulting foam. The pharmaceutically acceptable acids can also provide bactericidal effects.

Accordingly, the present invention provides a method of forming a polyurethane foam suitable for use as a wound-contacting layer, said method comprising: forming a prepolymer mixture by mixing 1 part weight of an isocyanate-capped prepolymer having from 0.5 to 4.8 meq. NCO groups/g,from 0.4 to 1.0 parts by weight of water, and from 0.05 to 2.0 meq H⁺/g of the prepolymer mixture of a nonvolatile pharmaceutically acceptable acid other than an amino acid, wherein the nonvolatile pharmaceutically acceptable acid is dissolved in said water prior to admixture with said prepolymer; and allowing said prepolymer mixture to cure to form a foamed product; followed by drying the foamed product.

Preferably, the isocyanate - capped prepolymer has from 0.5 to 1.2 meq. NCO groups/g, for the reasons set forth above.

Preferably, the step of mixing is carried out in the presence of from 0.05 to 0.4 parts by weight of a C₁ to C₃ monohydric alcohol. the step of drying then results in a product containing less than 1% by weight of the monohydric alcohol.

Preferably, the nonvolatile pharmaceutically acceptable acid is selected from the group consisting of ascorbic acid, monocarboxylic acids such as stearic acid or lactic acid, dicarboxylic acids such as citric acid, sorbic acid, malic acid or tartaric acid, or polycarboxylic acids such as edetic acid, alginic acid or hyaluronic acid. Mixtures of any of these acids may also be used. The preferred pharmaceutically acceptable acids are ascorbic acid and hyaluronic acid. The pharmaceutically acceptable acid is not an amino acid.

The nonvolatile pharmaceutically acceptable acid is present in the prepolymer mixture in an amount of from 0.05 to 2.0 meq H⁺/g,preferably from 0.1 to 1.0 meq H⁺/g. This corresponds roughly to generally about 1% to 20% by weight, preferably 2% to 10% by weight of the nonvolatile pharmaceutically acceptable acid, based on the weight of the prepolymer mixture, for a typical organic acid such as ascorbic acid.

The nonvolatile pharmaceutically acceptable acid is dissolved in the water component prior to combination with the polyurethane prepolymer to form the prepolymer mixture. Preferably, the amount and acid strength (pKₐ) of the nonvolatile pharmaceutically acceptable acid are such that the acid is present in an amount sufficient to lower the pH of the water components to less than 4.0, preferably to less than 3.0, and most preferably to less than 2.5. The acid amount and strength should nevertheLess be sufficiently low to avoid stinging when the foam is applied to the wound.

The use of a relatively small amount of water in accordance with the present invention produces an initial reaction mixture of much higher initial viscosity. Carbon dioxide formed by hydrolysis of isocyanate end groups is therefore trapped, producing a foamed hydrogel.

The present invention also provides a polyurethane foam which is suitable for use as a wound-contacting layer, the foam having a density of at least 0.28 g/cm³, wherein the foam contains less than 1% by weight of water-soluble alcohols, and the foam contains at least 4% by weight of one or more nonvolatile pharmaceutically acceptable acids other than an amino acid.

Preferably, the amount and composition of the entrapped nonvolatile pharmaceutically acceptable acids in the foam are as hereinbefore defined. The presence of acids in a wound contacting foam can promote wound healing, both through the bactericidal and antiseptic effects of the acid, and through particular wound healing properties of certain acids. Preferably, the polyurethane foam according to the present invention is obtainable by a process according to the present invention.

Foams produced according to the method of the invention have a density of at least 0.28 g/cm³, and preferably at least 0.30 g/cm³. Particularly preferred foams have a density in the range 0.3 to 1.0 g/cm³, e.g. about 0.5 g/cm³.

The foams of the invention also preferably have an elongation at break of at least 150%, and more preferably at least 300%. Particularly preferred foams according to the invention have an elongation at break in the range from 500 to 2000%.

Depending on the proportions of other additives, the foams of the invention have an absorbency of at least 3 g saline/g, preferably at least 5 g/g, and more preferably from 8 to 20 g/g. The foams are thus highly absorbent, yet conformable.

The foams of the invention also have the property of swelling and expanding when water is absorbed. This is particularly advantageous in a wound contact layer, because the swelling of the foam causes it to move inwards towards the wound bed thus filling the wound cavity. This encourages the wound to heal from the base upwards and outwards, and it discourages epithelialization over the wound surface before the bed has been filled with granulation tissue.

The degree of swelling of the foams of the present invention on complete saturation with an aqueous medium is typically at least 100% (expressed in terms of increase in volume), and preferably at least 200%. Preferred foams swell by 400 to 800%. Despite this high degree of swelling, however the foams of the invention retain their integrity even after absorption of large quantities of water.

Moreover, the foams are found to have a morphology which is particularly appropriate for low adherence wound dressings. The foams are open-celled, the cells being very regular in size and shape, with very smooth edges to the pores in the walls of the cells. Typically, the cells of the foams of the invention have an average diameter in the - range 0.1 to 0.6 mm.

The prepolymer which is used in the method of the invention is preferably an isocyanate-capped polyether, such as an ethyleneoxy/propyleneoxy copolymer. A particularly suitable prepolymer is that available under Trade Mark HYPOL Hydrogel.

Although the preferred methods of the invention comprehend the use of any of methanol, ethanol or propanol, the use of methanol is particularly preferred. All three alcohols reduce the rate of reaction between the isocyanate-capped prepolymer and water, but the effect of methanol is more marked. A reduction of the reaction rate is desirable in order to facilitate mixing of the various components and spreading of the reaction mixture into a layer of suitable thickness for curing. In addition, the monohydric alcohol serves to end cap some of the NCO end groups, preventing reaction with water to form the urea linkage. This also gives a more flexible, conformable foam.

More preferably, one part by weight of the isocyanate-capped prepolymer is mixed with water in the presence of from 0.05 to 0.25 parts by weight of methanol or from 0.1 to 0.3 parts by weight of ethanol.

It will be appreciated that. other components may be added to the reaction mixture in the method of the invention, in order to give desired properties to the product. In particular, it is preferable to include a small proportion (e.g. up to 30% by weight of the wet composition) of a rubber, which may be either natural or synthetic. This has the effect of increasing the cure time for the polyurethane, and increases extensibility, strength and tack. Most importantly, it substantially reduces shrinkage of the gel on drying, and it also improves bubble - formation, producing more regular, smaller bubbles.

Preferably, the rubber is added in the form of a latex, ie. a suspension or emulsion of the rubber in an aqueous medium. The latex will generally comprise 40 to 70% solids by weight, e.g. 50 to 60% by weight. If the foam is to be used as a wound contact layer, the rubber must of course be pharmaceutically acceptable.

Acrylic-based rubbers are particularly preferred. These are commercially available in the form of latexes, such as PRIMAL B-15J and RHOPLEX N-560 (Registered Trade Marks), manufactured by the Rohm & Haas company.

In addition to the methanol or ethanol, other alcohols, and particularly polyols, may be included in the reaction mixture to produce a softer, more conformable foam. For example, a polyol sold by Bayer AG under the Registered Trade Mark Levagel may be used. However, traces of such alcohols are likely to remain in the free form after the foaming reaction, and these traces may be difficult to remove from the foam merely by heating. The use of higher boiling alcohols is therefore preferably avoided if the foam is to be used as a wound contact layer, because of the likelihood that such alcohols will be leached from the foam during use of the dressing. When used as or in wound dressings, the foams of the invention contain less than 1% by weight of water soluble alcohols, and more preferably less than 0.1% by weight. It is particularly preferred that the foams of the invention are essentially free of water soluble alcohols (e.g. less that 0.01% by weight).

For use as a wound-contact layer, the foams of the invention may also include topical medicaments and antiseptics, such as silver sulfadiazine, povidone iodine, chlorhexidine acetate and chlorhexidine gluconate, as well as other therapeutically useful additives such as polypeptide growth factors and enzymes.

The present invention also provides a wound dressing comprising a wound contact layer formed from a polyurethane foam as described above, in conjunction with a water-repellant or water-impermeable backing layer. It is greatly preferred that the backing layer also be moisture vapour permeable, as well as being extensible and conformable. A particularly suitable material is a high density polyurethane foam, such as MEDIFIX 4003 or 4005 (Registered Trade Mark). These are polyurethane foams of a blocked toluene diisocyanate nature, and are predominantly closed cell.

A particularly advantageous presentation for the dressing of the invention is as an island of wound-contact material on a backing layer, wherein at least the marginal portions of the backing layer are coated with adhesive. Any medically accepted, skin friendly adhesive is suitable, including acrylic, hydrocolloid, polyurethane and silicone based adhesives.

The adhesive may be applied either continuously or discontinuously over the marginal portions of the backing layer. Preferably, however, the adhesive is applied continuously over the whole of the backing layer if the backing layer is not itself impermeable to bacteria, so as to ensure that the backing layer/adhesive combination is impermeable to bacteria.

It is also preferred that the combination of adhesive and backing layer have a minimum moisture vapour permeability of 400 g/m²/24 hrs, and preferably at least 700 g/m²/24 hrs.

The preferred adhesive is a polyurethane gel material known as LEVAGEL (Registered Trade Mark) and marketed by Bayer AG. This adhesive is made up of three components, namely a modified diphenylmethane diisocyanate, high molecular weight polyhydroxy polyether and a catalyst (dibutyltindilaurate). These three components may be mixed such that the gel contains 4-10 parts (preferably 4.6-6.4 parts) of the modified diphenylmethane diisocyanate, 99.9-99.9975 parts, (preferably 99.94-99.995 parts) of the polyhydroxy polyether and 0.0025-0.1 parts (preferably 0.005-0.06 parts) of the catalyst.

The gel may be mixed by the methods given in U.S. Patent No. 4,661,099 and applied by conventional coating methods to the backing. The thickness of the gel layer may be between 0.001 mm and 1.0 mm, and preferably between 0.05 mm and 005 mm, giving a coating weight of between 25 g/m² and 250 g/m².

The dressing may also contain a wicking layer between the wound contact layer and the backing layer. Such a wicking layer provides absorbency, but more importantly it encourages moisture to move from the wound facing side of the dressing to the back of the dressing where it escapes out of the dressing through the breathable backing. It should have good wicking properties so that moisture can be spread over as large a surface area as possible, thus increasing evaporation. The overall effect of this layer is to draw moisture from the wound facing layer, thus decreasing the chances of wound maceration, and to increase evaporation through the backing of the dressing.

The wicking layer may be formed of several plies (which may or may not be the same) if desired, but it is preferred that the total thickness of the wicking layer does not exceed 1 mm. It is also preferred that the wicking layer be substantially the same size and shape as the wound-facing layer, or slightly smaller than the wound-facing layer.

Suitable materials for the wicking layer include nonwoven, woven and knitted fabrics. Nonwoven viscose fabrics such as those conventionally used for making nonwoven surgical swabs are preferred, but it will be understood that many alternative fabrics (particularly other cellulosic fabrics) could be used in their place.

The dressings of the invention will generally be sterile and enclosed in a conventional bacteria-proof envelope. Sterilization may conventionally be carried out using γ-irradiation, but other sterilization methods such as electron beam sterilization may also be used.

The effect of addition of a nonvolatile acid on experimental curing rates of polyurethane foams for use in wound dressings is shown by the following specific embodiments, which are non-limiting examples for the purpose of illustration only.

### Example 1 (comparative)

Methanol (6g) was added to HYPOL (Registered Trade Mark) Hydrogel prepolymer (50g; NCO content 0.5-1.2 meq/g) in a disposable cup and mixed thoroughly for a few seconds. Water (32g) and Latex (12g) were then added to the HYPOL mixture and stirred vigorously. The foaming mixture was poured onto release paper and spread using a stainless steel hand spreader set at a gap of 2.2mm. The foam was left to cure and the foam sheet and release paper were placed in an oven (80°C - 100°C (30 min) to drive off the water. After cooling, the foam was lifted from the release paper, allowed to shrink, and replaced on the same paper. The foam was then kiss-cut to size and shape.

### Example 2

The procedure of Example 1 was repeated, but with the preliminary step of dispersing 0.5%, 1.0%, 5.0% and 10.0% by weight, based on the weight of the prepolymer mixture, of ascorbic acid in the water. The pH of the resulting aqueous solutions, and the measured cure times of the prepolymer mixtures, are given in Table 1.

### Example 3

The procedure of Example 1 was repeated, but with the preliminary step of dispersing 0.5%, 1.0%, 5.0%, 10.0% or 20.0% by weight, based on the weight of the prepolymer mixture, of lactic acid in the water. The pH of the resulting aqueous solutions, and the measured cure times of the prepolymer mixtures, are also given in Table 1.

### Example 4

The procedure of Example 1 was repeated, but with the preliminary step of dispersing 0.5%, 1.0%, 5.0%, 10.0% or 20.0% of malic acid, based on the weight of the prepolymer mixture, in the water. The pH of the resulting aqueous solutions, and the measured cure times of the prepolymer mixtures, are given in Table 1.

**Table 1**

| **Formulation** | **Acid %** | **Aqueous pH** | **Polymer cure time minutes** |
|---|---|---|---|
| Standard formulation | 0 | 6.01 | 2.0 |
| Standard formulation + ascorbic acid | 0.5 | 3.21 | 3.5 |
| | 1.0 | 2.98 | 6.5 |
| | 5.0 | 2.07 | 34.0 |
| | 10.0 | 1.79 | >150.0 |
| Standard formulation + lactic acid | 0.5 | 2.25 | 2.5 |
| | 1.0 | 1.85 | 2.5 |
| | 5.0 | 1.43 | 3.3 |
| | 10.0 | 1.23 | 4.5 |
| | 20.0 | 1.16 | 10.4 |
| Standard formulation + malic acid | 0.5 | 2.30 | 3.1 |
| | 1.0 | 1.99 | 2.6 |
| | 5.0 | 1.43 | 3.0 |
| | 10.0 | 1.06 | 3.3 |
| | 20.0 | 0.88 | > 150.0 |

It can be seen that the addition of acids to the prepolymer mixtures results in extended cure times. The extended cure times permit controlled spreading of the prepolymer mixtures to form foam layers for use in wound dressings. The effect of extended cure times at low concentrations is especially marked for ascorbic acid.

The water absorbency, density, and elongation at break of a number of foams manufactured in accordance with the invention as in the above Examples were measured, and the results were as shown in Table 2.

**Table 2**

| **Formulation** | **Acid %** | **Absorbency g/g** | **Density g/cm**^{**3**} | **Elongation at break %** |
|---|---|---|---|---|
| Standard Formulation | 0 | **12.0** | **0.30** | **872** |
| Standard formulation + ascorbic acid | 1 | 7.0 | 0.30 | 902 |
| | 5 | 9.1 | 0.60 | 987 |
| | **Average** | **8.1** | **0.45** | **947** |
| Standard formulation + lactic acid | 1 | 9.2 | 0.40 | 914 |
| | 5 | 7.5 | 0.64 | 740 |
| | **Average** | **8.4** | **0.52** | **827** |
| Standard formulation + malic acid | 1 | 11.8 | 0.42 | 953 |
| | 5 | 14.4 | 0.60 | 876 |
| | **Average** | **13.1** | **0.51** | **915** |
| Standard Formulation + tartaric acid | 1 | 7.6 | 0.27 | 907 |
| | 5 | 2.6 | 0.80 | 931 |
| | **Average** | **5.1** | **0.54** | **919** |
| Standard Formulation + citric acid | 1 | 11.9 | 0.28 | 865 |
| | 5 | 11.3 | 0.40 | 1025 |
| | **Average** | **11.6** | **0.34** | **945** |
| Standard Formulation + alginic acid | 1 | 11.0 | 0.40 | 900 |
| | 5 | 6.5 | 0.91 | 716 |
| | **Average** | **8.8** | **0.66** | **808** |

It can thus be seen that the incorporation of acid has little adverse effect on the absorbency or elongation at break of the foams.

It should also be noted that the acids in the foams according to the present invention have beneficial therapeutic effects, including bactericidal effects, especially.for ascorbic acid.

The above embodiments have been described by way of example only. Many other embodiments of the present invention falling within the scope of the accompanying claims will be apparent to the skilled reader.

## Claims

1. A method of forming a polyurethane foam suitable for use as a wound-contacting layer, said method comprising:
forming a prepolymer mixture by mixing
1 part weight of an isocyanate-capped prepolymer having from 0.5 to 4.8 meq. NCO groups/g,
from 0.4 to 1.0 parts by weight of water, and
from 0.05 to 2.0 meq H⁺/g of the prepolymer mixture of a nonvolatile pharmaceutically acceptable acid other than an amino acid,
wherein the nonvolatile pharmaceutically acceptable acid is dissolved in said water prior to admixture with said prepolymer; and
allowing said prepolymer mixture to cure to form a foamed product; followed by
.drying the foamed product.

2. A method according to claim 1, wherein the isocyanate - capped prepolymer has from 0.5 to 1.2 meq. NCO groups/g.

3. A method according to claim 1 or 2, wherein the step of mixing is carried out in the presence of from 0.05 to 0.4 parts by weight of a C₁ to C₃ monohydric alcohol.

4. A method according to claim 3, wherein the monohydric alcohol is methanol.

5. A method according to any preceding claim, wherein the nonvolatile pharmaceutically acceptable acid is selected from the group consisting of ascorbic acid, monocarboxylic acids, such as stearic acid or lactic acid, dicarboxylic acids such as sorbic acid, citric acid, malic acid or tartaric acid, or polycarboxylic acids such as edetic acid, alginic acid, hyaluronic acid and mixtures thereof.

6. A method according to any preceding claim, wherein the nonvolatile pharmaceutically acceptable acid is present in said prepolymer mixture in an amount of from 0.1 to 1.0 meq H⁺/g.

7. A method according to any preceding claim, wherein the pharmaceutically acceptable acid is present in an amount sufficient to lower the pH of said water to less than 4.0

8. A method according to claim 8, wherein the nonvolatile pharmaceutically acceptable acid is present in an amount sufficient to lower the pH of said water to less than 3.0.

9. A method according to any preceding claim, wherein said prepolymer mixture contains from 1% to 20% by weight of said nonvolatile pharmaceutically acceptable acid.

10. A method according to any preceding claim, wherein the isocyanate-capped prepolymer is an isocyanate-capped polyether prepolymer.

11. A method according to claim 11 wherein the isocyanate-capped polyether prepolymer is an isocyanate-capped ethyleneoxy/propyleneoxy copolymer.

12. A method according to any preceding claim wherein one part by weight of the isocyanate-capped prepolymer is mixed with from 0.6 to 0.9 parts by weight of water.

13. A method according to any preceding claim wherein one part by weight of the isocyanate-capped prepolymer is mixed with water in the presence of from 0.05 to 0.25 parts by weight of methanol or from 0.1 to 0.3 parts by weight of ethanol.

14. A polyurethane foam which is suitable for use as a wound-contacting layer, said foam having a density of at least 0.28 g/cm³, said foam containing less that 1% by weight of water-soluble alcohols, and said foam further containing at least 4% by weight of one or more nonvolatile pharmaceutically acceptable acids other than an amino acid.

15. A polyurethane foam according to claim 14 having a density in the range 0.32 to 0.48 g/cm³.

16. A polyurethane foam according to claim 14 having an elongation at break of at least 150%.

17. A polyurethane foam according to any of claims 14 to 16 having an elongation at break in the range from 500 to 2000%.

18. A polyurethane foam according to any of claims 14 to 17 having an absorbency of at least 3 g saline/g.

19. A polyurethane foam according to any of claims 14 to 18 having a swellability on absorption of water of at least 200% by volume.

20. A foam according to any of claims 14 to 19, containing less than 0.01% by weight of water-soluble alcohols.

21. A wound dressing having a wound-contact layer formed from a polyurethane foam according to any of claims 14 to 20.

## Patentansprüche

1. Verfahren zum Formen eines Polyurethan-Schaums, geeignet für die Verwendung als eine Wunden-kontaktierende Schicht, besagtes Verfahren umfassend:
Formen einer Präpolymer-Mischung durch Mischen von
einem Gewichtsteil eines Isocyanat-gekappten Präpolymers, das von 0,5 bis 4,8 meq
NCO-Gruppen/g hat,
von 0,4 bis 1,0 Gewichtsteilen Wasser und
von 0,05 bis 2,0 meq H⁺/g der Präpolymer-Mischung einer nicht flüchtigen, pharmazeutisch akzeptablen Säure, die nicht eine Aminosäure ist,
worin die nicht flüchtige, pharmazeutisch akzeptable Säure in besagtem Wasser vor dem Mischen mit besagtem Präpolymer gelöst ist; und
Erlauben besagter Präpolymer-Mischung zu vulkanisieren, um ein geschäumtes Produkt zu bilden; gefolgt von
Trocknen des geschäumten Produkts.

2. Verfahren gemäß Anspruch 1, worin das Isocyanat-gekappte Präpolymer von 0,5 bis 1,2 meq NCO-Gruppen /g hat.

3. Verfahren gemäß der Ansprüche 1 oder 2, worin der Schritt des Mischens in der Anwesenheit von 0,05 bis 0,4 Gewichtsteilen eines C₁ bis C₃ monohydrischen Alkohols durchgeführt wird.

4. Verfahren gemäß Anspruch 3, worin der monohydrische Alkohol Methanol ist.

5. Verfahren gemäß einem der vorhergehenden Ansprüche, worin die nicht flüchtige, pharmazeutisch akzeptable Säure ausgewählt ist aus der Gruppe bestehend aus Ascorbinsäure, Monocarbonsäuren, wie Stearinsäure oder Milchsäure, Dicarbonsäuren, wie Sorbinsäure, Zitronensäure, Apfelsäure oder Weinsäure, oder Polycarbonsäuren, wie Edetinsäure, Alginsäure, Hyaluronsäure und Mischungen davon.

6. Verfahren gemäß einem der vorhergehenden Ansprüche, worin die nicht flüchtige, pharmazeutisch akzeptable Säure in besagter Präpolymer-Mischung in einer Menge von 0,1 bis 1,0 meq H⁺/g anwesend ist.

7. Verfahren gemäß einem der vorhergehenden Ansprüche, worin die pharmazeutisch akzeptable Säure in einer Menge anwesend ist, die ausreicht, den pH des besagten Wassers auf weniger als 4,0 zu senken.

8. Verfahren gemäß Anspruch 7, worin die nicht flüchtige, pharmazeutisch akzeptable Säure in einer Menge anwesend ist, die ausreicht, den pH des besagten Wassers auf weniger als 3,0 zu senken.

9. Verfahren gemäß einem der vorhergehenden Ansprüche, worin besagte Präpolymer-Mischung von 1 bis 20 Gew.-% von besagter nicht flüchtiger, pharmazeutisch akzeptabler Säure enthält.

10. Verfahren gemäß einem der vorhergehenden Ansprüche, worin das Isocyanat-gekappte Präpolymer ein Iscyanat-gekapptes Polyether-Präpolymer ist.

11. Verfahren gemäß Anspruch 10, worin das Isocyanat-gekappte Polyether-Präpolymer ein Isocyanat-gekapptes Ethylenoxy-Propylenoxy-Kopolymer ist.

12. Verfahren gemäß einem der vorhergehenden Ansprüche, worin ein Gewichtsteil des Isocyanat-gekappten Präpolymers mit von 0,6 bis 0,9 Gewichtsteilen Wasser gemischt wird.

13. Verfahren gemäß einem der vorhergehenden Ansprüche, worin ein Gewichtsteil des Isocyanat-gekappten Präpolymers mit Wasser in der Anwesenheit von 0,05 bis 0,25 Gewichtsteilen Methanol oder von 0,1 bis 0,3 Gewichtsteilen Ethanol gemischt wird.

14. Polyurethan-Schaum, welcher für die Verwendung als eine Wunden-kontaktierende Schicht geeignet ist, wobei besagter Schaum eine Dichte von mindestens 0,28 g/cm³ hat, besagter Schaum weniger als 1 Gew.-% wasserlösliche Alkohole enthält und besagter Schaum weiterhin mindestens 4 Gew.-% einer oder mehrerer nicht flüchtiger, pharmazeutisch akzeptabler Säuren, die nicht Aminosäuren sind, enthält.

15. Polyurethan-Schaum gemäß Anspruch 14, der eine Dichte im Bereich von 0,32 bis 0,48 g/cm³ hat.

16. Polyurethan-Schaum gemäß Anspruch 14, der eine Bruchdehnung von mindestens 150 % hat.

17. Polyurethan-Schaum gemäß einem der Ansprüche 14 bis 16, der eine Bruchdehnung im Bereich von 500 bis 2000 % hat.

18. Polyurethan-Schaum gemäß einem der Ansprüche 14 bis 17, der ein Absorptionsvermögen von mindestens 3 g Saline/g hat.

19. Polyurethan-Schaum gemäß einem der Ansprüche 14 bis 18, der eine Quellfähigkeit nach Absorption von Wasser von mindestens 200 Volumen-% hat.

20. Schaum gemäß einem der Ansprüche 14 bis 19, beinhaltend weniger als 0,01 Gew.-% wasserlösliche Alkohole.

21. Wund-Verbandstoff, der eine Wunden-kontaktierende Schicht, geformt aus einem Polyurethan-Schaum gemäß einem der Ansprüche 14 bis 20, hat.

## Revendications

1. Procédé pour la formation d'une mousse de polyuréthane qui convient pour être utilisée comme couche de contact avec une lésion, lequel procédé comprend :
■ la formation d'un mélange prépolymère en mélangeant
■ 1 partie en poids d'un prépolymère à coiffe isocyanate possédant de 0,5 à 4,8 meq . groupes NCO/g,
■ de 0,4 à 1,0 partie en poids d'eau, et
■ de 0,05 à 2,0 meq H⁺ / g du mélange prépolymère d'un acide non volatil pharmaceutiquement acceptable autre qu'une acide aminé,
dans lequel l'acide non volatil pharmaceutiquement acceptable est dissous dans ladite eau avant le mélange avec ledit prépolymère ; et
laisser ledit mélange prépolymère durcir pour former une mousse ; et ensuite
sécher la mousse.

2. Procédé selon la revendication 1, dans lequel le prépolymère à coiffe isocyanate possède de 0,5 à 1,2 meq. groupes NCO/g.

3. Procédé selon la revendication 1 ou 2, dans lequel l'étape de mélange s'effectue en présence de 0,05 à 0,4 partie en poids d'un alcool monohydroxylé en C₁ à C₃.

4. Procédé selon la revendication 3, dans lequel l'alcool monohydroxylé est le méthanol.

5. Procédé selon l'une quelconque des revendications précédentes , dans lequel l'acide non volatil pharmaceutiquement acceptable est choisi dans le groupe consistant en l'acide ascorbique, les acides monocarboxyliques tels que l'acide stéarique ou l'acide lactique, les acides dicarboxyliques tels que l'acide sorbique, l'acide citrique, l'acide malique ou l'acide tartrique, ou les acides polycarboxyliques tels que l'acide édétique, l'acide alginique, l'acide hyaluronique et leurs mélanges.

6. Procédé selon l'une quelconque des revendications précédentes, dans lequel l'acide non volatil pharmaceutiquement acceptable est présent dans ledit mélange prépolymère en une quantité de 0,1 à 1,0 meq H⁺ /g.

7. Procédé selon l'une quelconque des revendications précédentes, dans lequel l'acide non volatil pharmaceutiquement acceptable est présent en une quantité suffisante pour abaisser le pH de ladite eau à moins de 4,0.

8. Procédé selon la revendication 7, dans lequel l'acide non volatil pharmaceutiquement acceptable est présent en une quantité suffisante pour abaisser le pH de ladite eau à moins de 3,0.

9. Procédé selon l'une quelconque des revendications précédentes, dans lequel ledit mélange prépolymère contient de 1 % à 20 % en poids dudit acide non volatil pharmaceutiquement acceptable.

10. Procédé selon l'une quelconque des revendications précédentes, dans lequel le prépolymère à coiffe isocyanate est un prépolymère polyéther à coiffe isocyanate.

11. Procédé selon la revendication 10, dans lequel le prépolymère polyéther à coiffe isocyanate est un copolymère oxyde d éthylène / oxyde de propylène à coiffe isocyanate.

12. Procédé selon l'une quelconque des revendications précédentes, dans lequel une partie en poids du prépolymère à coiffe isocyanate est mélangée avec de 0,6 à 0,9 partie en poids d'eau.

13. Procédé selon l'une quelconque des revendications précédentes, dans lequel une partie en poids du prépolymère à coiffe isocyanate est mélangée avec l'eau en présence de 0,05 à 0,25 partie en poids de méthanol ou de 0,1 à 0,3 partie en poids d'éthanol.

14. Mousse de polyuréthane qui convient pour être utilisée comme couche de contact avec une lésion, ladite mousse ayant une masse volumique d'au moins 0,28 g/cm³ , ladite mousse contenant moins de 1 % en poids d'alcools solubles dans l'eau et ladite mousse contenant en outre au moins 4 % en poids d'un ou plusieurs acides non volatils pharmaceutiquement acceptables autres qu'un acide aminé.

15. Mousse de polyuréthane selon la revendication 14, possédant une masse volumique comprise dans l'intervalle de 0,32 à 0,48 g/cm³.

16. Mousse de polyuréthane selon la revendication 14, possédant un allongement à la rupture d'au moins 150 %.

17. Mousse de polyuréthane selon l'une quelconque des revendications 14 à 16 possédant un allongement à la rupture compris dans l'intervalle de 500 à 2000%.

18. Mousse de polyuréthane selon l'une quelconque des revendications 14 à 17, possédant un pouvoir absorbant d'au moins 3 g de solution saline/g.

19. Mousse de polyuréthane selon l'une quelconque des revendications 14 à 18, possédant une aptitude à gonfler lors de l'absorption d'eau d'au moins 200 % en volume.

20. Mousse selon l'une quelconque des revendications 14 à 19, contenant moins de 0,01 % en poids d'alcools solubles dans l'eau.

21. Pansement possédant une couche de contact avec la lésion formé à partir d'une mousse de polyuréthane selon l'une quelconque des revendications 14 à 20.
